# EUROPEAN PATENT APPLICATION

(11) **EP 2 939 705 A1**
(43) Date of publication of application: **04.11.2015**
(21) Application number: 12890678.1
(22) Date of filing: 25.12.2012
(51) Int. Cl.: A61N 1/30

(54) **IONTOPHORESIS PATCH**

(71) Applicant: Terumo Kabushiki Kaisha, Tokyo 151-0072 (JP); Teikoku Seiyaku Co., Ltd., Higashikagawa-shi, Kagawa 769-2695 (JP)
(72) Inventor: YAEGASHI, Mitsutoshi, Ashigarakami-gun Kanagawa 259-0151 (JP); HASUI, Akihiro, Higashikagawa-shi Kagawa 769-2695 (JP)
(74) Representative: Casalonga
(86) International application number: PCT/JP2012/083431
(87) International publication number: WO 2014/102896

(57) **Abstract**

An iontophoresis patch (12) which outputs an electric current from an energizing device (14) to an external conductor comprises: a donor portion (16) that has a first contact member (34); a reference portion (18) that has a second contact member (38) and is located on the external conductor away from the donor portion (16); and an electrode body (20) that has a first electrode (42, 70, 80, 90) and a second electrode (44) and that extends across the donor portion (16) and the reference portion (18). The electrode body (20) has a single-sided wiring structure in which the first electrode (42, 70, 80, 90) and the second electrode (44) are formed by a conductive ink being printed on one side of a film (28), the printing pattern of the first electrode (42) has a gap area (43, 71, 81, 91) in which the conductive ink is not printed, and the film (28) and the first contact member (34) are transparent.

## Description

### Technical Field

The present invention relates to an iontophoresis patch for use in a transdermal drug delivery device, which is based on the principles of iontophoresis wherein a drug penetrates into the skin of a human being by passing a low electric current through the skin.

### Background Art

One process for transdermal drug delivery is referred to as iontophoresis. According to iontophoresis, electrodes are applied to two spaced locations on a skin, and an electric current is caused to flow from one of the electrodes across a stratum corneum through the skin to the other electrode, thereby moving a drug that has been charged in the stratum corneum into the skin on the basis of the principles of electrophoresis for transdermal drug absorption. Iontophoresis serves as a basis for one of transdermal drug delivery systems.

One of the two electrodes is held in contact with a gel containing the drug, and is called a donor section. The other electrode is held in contact with a gel containing a salt solution, and is called a reference section. In principle, although the charged drug is a target whose absorption is to be promoted, it has been reported that since a water flow is developed by the flowing electric current, drugs that are free of charges and drugs that have a large molecular weight have an increased skin permeability.

General iontophoresis drug delivery systems have a patch containing a drug and a controller for supplying an electric current to the patch. Heretofore, drug delivery systems which employ a commercial power supply (e.g., AC 100 V) for supplying an electric current are in general use and large in size. However, as disclosed in Japanese Laid-Open Patent Publication No. 10-512474 (PCT) and Japanese Laid-Open Patent Publication No. 2012-139293, there have been developed portable drug delivery systems wherein a small-size controller with a cell for supplying an electric current is mounted on a patch having a donor section and a reference section, in order not to limit the actions of a patient while a drug is being delivered to the patient.

### Summary of Invention

The electrodes referred to above are formed, for example, by printing a film of PET (polyester), PI (polyimide), or the like with an electrically conductive ink. While the gel at the donor section and the film can be made transparent, because the electrically conductive ink is not transparent, it has heretofore been impossible for the user to visually confirm through the patch a body region into which the drug penetrates, and it has been inconvenient for the user to apply the patch to the patient.

It is an object of the present invention to provide an iontophoresis patch which can easily be applied to a patient at a desired position.

According to the present invention, there is provided an iontophoresis patch including a first contact member and a second contact member configured to be held in contact with an external conductor and configured to supply an electric current from an electric energizer to the external conductor, comprising a donor section including the first contact member, the first contact member containing a drug to penetrate into the external conductor, a reference section including the second contact member, the reference section being placed on the external conductor and being spaced from the donor section, and an electrode body having a first electrode and a second electrode configured to supply an electric current from the electric energizer to the first contact member and second contact member, the electrode body extending over the donor section and the reference section, wherein the electrode body is of a one-sided wiring structure having the first electrode and the second electrode which are formed by printing one surface of a flexible film in an electrically conductive ink, the first electrode has a printed pattern including a gap which is devoid of the electrically conductive ink, and the flexible film and the first contact member are transparent.

With the above arrangement, since the printed pattern of the first electrode includes a gap which is devoid of the electrically conductive ink, and the film and the first contact member are transparent, the region of the external conduction into which the drug penetrates can visually be recognized from a surface of the iontophoresis patch which is opposite to the first contact member. Therefore, the iontophoresis patch can be applied to the external conductor so that the first contact member is held in contact with the region of the external conductor.

In the above iontophoresis patch, the printed pattern of the first electrode is of a shape having at least either concentric circular wires or straight wires extending centrally through the donor section, the straight wires functioning as aiming lines for indicating a center of the first electrode. Therefore, the iontophoresis patch can be applied to the external conductor so that the center of the first contact member is held in contact with the region of the external conductor into which the drug penetrates.

In the iontophoresis patch, the printed pattern of the first electrode is of a spiral shape. Therefore, the iontophoresis patch can be applied to the external conductor so that the center of the first contact member is held in contact with the region of the external conductor into which the drug penetrates.

In the iontophoresis patch, the printed pattern of the first electrode is of at least a mesh shape. Therefore, the iontophoresis patch can be applied to the external conductor so that the center of the first contact member is held in contact with the region of the external conductor into which the drug penetrates.

In the iontophoresis patch, the printed pattern of the first electrode is of a radial shape. Therefore, the iontophoresis patch can be applied to the external conductor so that the center of the first contact member is held in contact with the region of the external conductor into which the drug penetrates.

According to the present invention, since the printed pattern of the first electrode includes a gap which is devoid of the electrically conductive ink, and the film and the first contact member are transparent, the region of the external conduction into which the drug penetrates can visually be recognized from a surface of the iontophoresis patch which is opposite to the first contact member. Therefore, the iontophoresis patch can be applied to the external conductor so that the first contact member is held in contact with the region of the external conductor.

### Brief description of Drawings

FIG. 1 is a perspective view showing the overall structure of a transdermal drug delivery device based on iontophoresis according to an embodiment of the present invention;
FIG. 2 is an exploded perspective view of an iontophoresis patch shown in FIG. 1;
FIG. 3A is a bottom view of an electrode film before a reference section thereof shown in FIG. 2 is folded over on itself;
FIG. 3B is a bottom view of the electrode film shown in FIG. 3A with an insulative resist placed on its surface;
FIG. 4A is a bottom view of the electrode film shown in FIG. 3A after the reference section has been folded over on itself;
FIG. 4B is a plan view of the electrode film shown in FIG. 4A;
FIG. 5 is a bottom view of an electrode film according to Modification 1 before a reference section thereof is folded over on itself;
FIG. 6 is a bottom view of an electrode film according to Modification 2 before a reference section thereof is folded over on itself; and
FIG. 7 is a bottom view of an electrode film according to Modification 3 before a reference section thereof is folded over on itself.

### Description of Embodiments

Iontophoresis patches according to preferred embodiments of the present invention will be described in detail below with reference to the accompanying drawings.

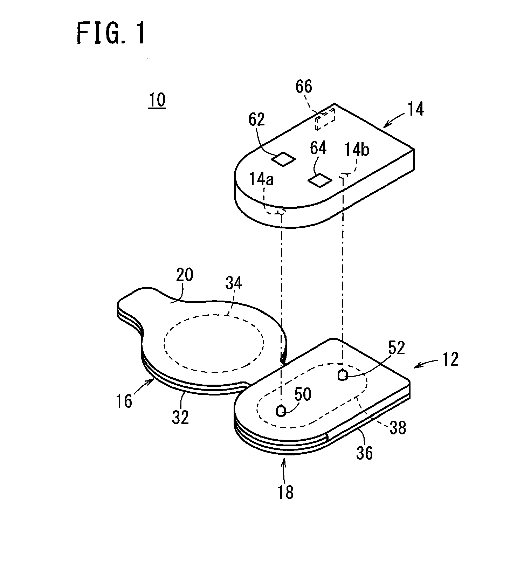

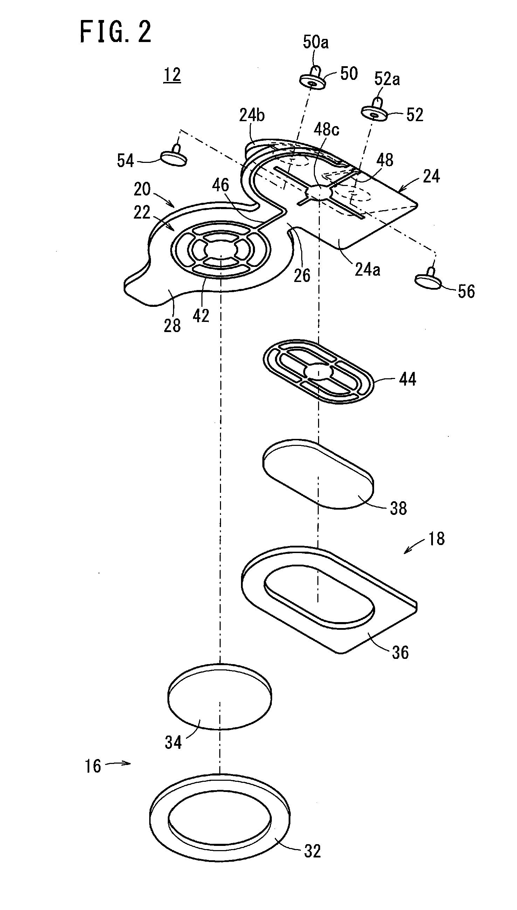

FIG. 1 shows in perspective the overall structure of a transdermal drug delivery device 10 according to an embodiment of the present invention. The transdermal drug delivery device 10 includes an iontophoresis patch 12 and an electric energizer (controller) 14 which are shown as being separate from each other. FIG. 2 shows in exploded perspective the iontophoresis patch 12 shown in FIG. 1.

The transdermal drug delivery device 10 (hereinafter referred to as "device 10") is a medical instrument for delivering and having a local anesthetic, e.g., an ionic anesthetic containing lidocaine, penetrate into an arm of a hemodialysis patient to relieve the pain of the patient when punctured with the dialysis needle. Specifically, the iontophoresis patch 12 (hereinafter referred to as "patch 12") is applied to the skin of the patient, which serves as an external electric conductor, and the electric energizer 14 supplies an electric current to the patch 12 to have the ionic anesthetic that is sealed in the patch 12 penetrate into the body of the patient. The patch 12 may be used with a device for delivering an ionic drug other than the ionic anesthetic to a patient, or may be used with a device for delivering a nonionic drug to a patient.

As shown in FIGS. 1 and 2, the device 10 includes the patch 12 and the electric energizer 14 that is placed on and electrically connected to the surface (upper surface) of the patch 12.

The patch 12 includes a donor section 16 in the form of a circular thin plate, a reference section 18, in the form of a rectangular thin plate having an arcuate side spaced from the donor section 16, and an electrode film (electrode body) 20 disposed over the donor section 16 and the reference section 18 and connected to the electric energizer 14. The electrode film 20 has a donor region 22 and a reference region 24 which are shaped correspondingly to the donor section 16 and the reference section 18, and a narrow bridge region 26 interconnecting the donor section 16 and the reference section 18 (see FIG. 2).

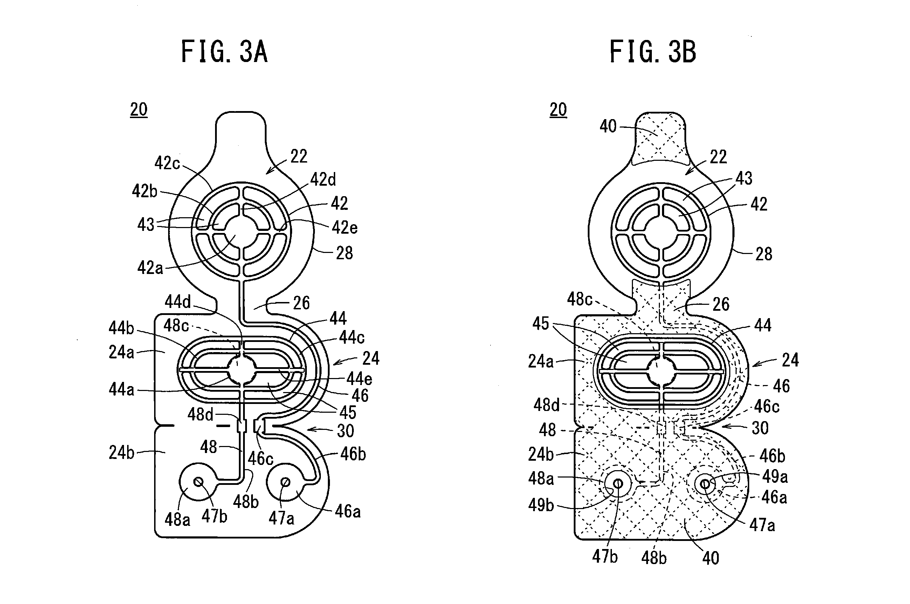

The electrode film 20 includes a base 28 that defines the outer profile of the donor region 22 and the reference region 24. The base 28 is of a single-layer structure over the donor region 22 and of a double-layer structure (double-layer lamination structure) over the reference region 24 where the base 28 is folded over on itself about a fold (bend) 30 (see FIGS. 3A and 3B). Specifically, the reference region 24 includes a first reference region 24a that serves as a side (bottom side) to be applied to the skin and a second reference region 24b folded back about the fold 30 so as to superpose on the reverse side of the first reference region 24a.

The donor section 16 has a circular donor pad 32 whose outer profile defines the outer profile of the donor section 16 and a transparent donor gel (first contact member) 34 that fills an opening formed in the donor pad 32. The donor region 22 of the electrode film 20 is electrically connected to the surface (upper surface in FIG. 2) of the donor gel 34. The reference section 18 has a rectangular reference pad 36 with an arcuate side, whose outer profile defines the outer profile of the reference section 18, and a transparent reference gel (second contact member) 38 that fills an opening formed in the reference pad 36. The reference region 24 of the electrode film 20 is electrically connected to the surface (upper surface in FIG. 2) of the reference gel 38.

Each of the donor pad 32 and the reference pad 36 is a sticky elastic member that sticks to a skin such as a human skin or the like with a certain degree of strength, and is insulative. Each of the donor pad 32 and the reference pad 36 may be made of a transparent material. The donor gel 34 is filled with the ionic anesthetic referred to above, whereas the reference gel 38 is filled with a liquid or solution containing an electrolytic substance, e.g., buffer salt, dietary salt, or the like that is not harmful to living bodies. Since a medical service worker who is familiar with needling finds it easy to puncture an area of 2.5 cm² on the patient's arm to be punctured for drug delivery, the contact surface (lower surface in FIG. 2) of the donor gel 34 and the reference gel 38 for contact with the skin may be in a range from 2.5 to 5.0 cm², for example. The donor gel 34 is placed in the opening in the donor pad 32, and the reference gel 38 is placed in the opening in the reference pad 36. When the donor pad 32 and the reference pad 36 are applied to the skin of the patient, the donor section 16 and the reference section 18 are substantially simultaneously brought into contact with the skin. Therefore, the patch 12 can simply be applied to the skin in one operation. The contact surface of the donor gel 34 and the reference gel 38 for contact with the skin may be sticky.

FIG. 3A is a bottom view of the electrode film 20 before the reference region 24 is folded over on itself, and FIG. 3B is a bottom view of the electrode film 20 shown in FIG. 3A with an insulative resist 40 placed on its surface. Specifically, FIG. 3A is a bottom view showing the electrode film 20 with the resist 40 (shown broken-line cross-hatched in FIG. 3B) being omitted from illustration. FIGS. 3A and 3B illustrate, in a bottom view, most of the surface area of the electrode film 20, which is provided by the donor region 22 and the first reference region 24a and serves as a bottom surface to be applied to the skin. In an actual product form, the second reference region 24b is folded back about the fold 30 provides an upper surface on which the electric energizer 14 is placed.

As shown in FIG. 3A, before the reference region 24 is folded over on itself, i.e., while the patch 12 is being fabricated, the electrode film 20 is in the form of a flexible board with the single base 28 where the reference region 24 is substantially symmetrical in shape with respect to the fold 30. The base 28 comprises a transparent thin soft film of resin such as polyester, polyimide, or the like.

The electrode film 20 is of a one-sided wiring structure having electrodes and wires of the donor region 22 and the reference region 24 disposed on the surface of the base 28 before it is folded over on itself about the fold 30.

The donor region 22 includes a first electrode 42 having a circular profile that is disposed on the bottom surface of the base 28 (the lower surface of the donor region 22 in FIG. 2) and which is held in contact with and electrically connected to the donor gel 34. The reference region 24 includes a second electrode 44 having a substantially oblong profile that is disposed on the bottom surface of the base 28 (the bottom surface of the first reference region 24a in FIG. 2). The first electrode 42 and the second electrode 44 are electrically connected to the electric energizer 14 respectively through a first connection terminal wire 46 and a second connection terminal wire 48 that are disposed on the bottom surface of the base 28.

The first connection terminal wire 46 has a terminal base 46a disposed on the surface of the second reference region 24b, and a connection wire 46b extending tortuously from the terminal base 46a over the surface of the second reference region 24b and the surface of the bridge region 26 and connected to the first electrode 42. The terminal base 46a has a small-diameter hole 47a formed centrally therein which extends thicknesswise through the terminal base 46a and the base 28. The connection wire 46b extends along the arcuate sides of the second reference region 24b and the first reference region 24a, and also centrally through the bridge region 26, and is connected to the first electrode 42.

The second connection terminal wire 48 has a terminal base 48a so as to be juxtaposed to the terminal base 46a disposed on the surface of the second reference region 24b, and a connection wire 48b extending tortuously from the terminal base 48a to a substantially central area of the first reference region 24a. A circular electrode pad 48c (see FIG. 2) is disposed on the end portion of the connection wire 48b remote from the terminal base 48a. The second electrode 44 is held in electric contact with the electrode pad 48c. As with the terminal base 46a, the terminal base 48a has a small-diameter hole 47b formed centrally therein which extends thicknesswise through the terminal base 48a and the base 28. The connection wire 48b extends from the terminal base 48a to a central area of the second reference region 24b and then to a central area of the first reference region 24a where the connection wire 48b is connected to the electrode pad 48c.

The electrode film 20 is fabricated as follows: As shown in FIG. 3A, the surface of the base 28 before the reference region 24 is folded over on itself is printed with an electrically conductive ink containing silver to form the first electrode 42, the first connection terminal wire 46, and the second connection terminal wire 48. After the electrode pad 48c is formed on the second connection terminal wire 48, the upper surface of the electrode pad 48c is printed with an electrically conductive ink containing silver/silver chloride to form the second electrode 44. The second electrode 44 may be made of the same material as the first electrode 42. If the second electrode 44 is made of the same material as the first electrode 42, then the electrode pad 48c may be dispensed with, and the first electrode 42, the first connection terminal wire 46, the second connection terminal wire 48, and the second electrode 44 may be formed simultaneously.

The first electrode 42 and the second electrode 44 are formed by applying the electrically conductive ink in patterns including gaps to areas where the first electrode 42 and the second electrode 44 are formed. Specifically, the patterns in which the first electrode 42 and the second electrode 44 are printed have a plurality of gaps 43, 45 which are devoid of the electrically conductive ink. According to the present embodiment, the printed pattern of the first electrode 42 is made up of three concentric circular wires 42a, 42b, 42c and two straight wires 42d, 42e extending through the centers of the concentric circular wires 42a, 42b, 42c and being oriented perpendicularly to each other. Alternatively, the printed pattern of the first electrode 42 may be made up of any one of concentric circular wires and straight wires extending through the center of the donor section 16. The printed pattern of the second electrode 44 is made of a circular pad 44a and two substantial oblong wires 44b, 44c which are centrally aligned with each other, the oblong wires 44b, 44c being disposed around the circular pad 44a, and two straight wires 44d, 44e extending through the centers of the oblong wires 44b, 44c and being oriented perpendicularly to each other.

The donor gel 34 of the donor section 16 and the reference gel 38 of the reference section 18 are at least transparent, and the first electrode 42 and the second electrode 44 are formed by printing the transparent base 28 with the electrically conductive ink such that the gaps 43, 45 are included therein. Therefore, although those regions printed with the electrically conductive ink block applied light, the gaps 43, 45 do not block applied light, but are permeable to applied light. The donor gel 34 is disposed on the first reference region 24a such that the center of the donor gel 34 is held in substantial alignment with the concentric circular wires of the first electrode 42.

After the first electrode 42, the first connection terminal wire 46, the second connection terminal wire 48, and the second electrode 44 have been formed, the electrode film 20 is selectively sealed with the resist 40, which may be an insulative adhesive, paint, or the like, as shown in FIG. 3B. The resist 40 may be made of a transparent material. The resist 40 is not applied to the contact surfaces of the first electrode 42 and the second electrode 44 for contact with the donor gel 34 and the reference gel 38, and the resist 40 is not applied to circular holes 49a, 49b that extend respectively around the holes 47a, 47b and that are greater in diameter than the holes 47a, 47b. The electrically conductive ink may be applied in a plurality of printing processes to an increased wire thickness for reliably preventing electric conduction failures. The resist 40 may be replaced with an insulative sheet that covers the exposed surfaces of the wires in insulation.

The portions of the connection wires 46b, 48b of the first and second connection terminal wires 46, 48 which are aligned with the fold 30, i.e., the portions of the connection wires 46b, 48b which extend across the fold 30, are formed as widened portions 46c, 48d that are wider than the nearby portions of the connection wires 46b, 48b. When the first reference region 24a and the second reference region 24b are folded over on each other about the fold 30, the widened portions 46c, 48d prevent the connection wires 46b, 48b from being broken or turned up. As a result, the connection wires 46b, 48b are made highly durable and reliable at the time the reference region 24 is folded over on itself about the fold 30.

A process of fabricating the patch 12 using the electrode film 20 of the above constitution will be described below.

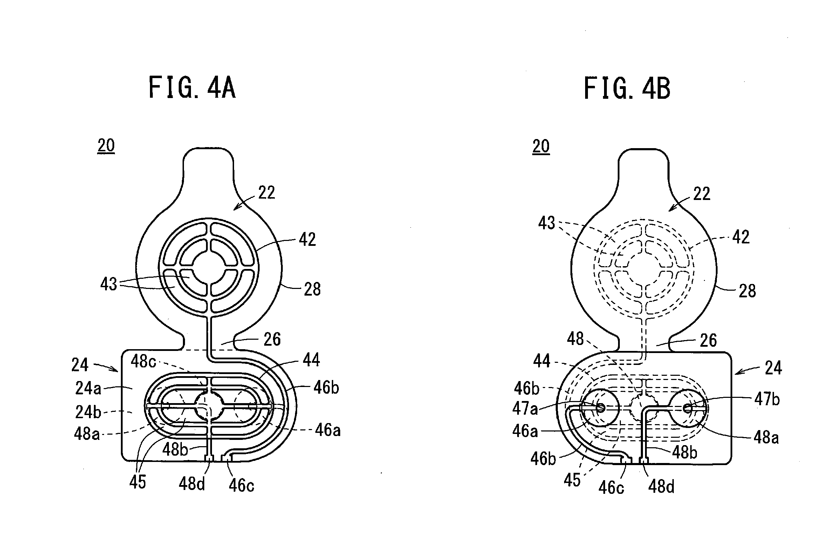

After the first electrode 42, the second electrode 44, the first connection terminal wire 46, and the second connection terminal wire 48 have been formed on the surface of the base 28, the reference region 24 is folded over on itself about the fold 30, as shown in FIGS. 4A and 4B. Then, the first reference region 24a and the second reference region 24b have their reverse sides kept in intimate contact with each other and bonded to each other by an adhesive or the like. In the reference region 24, therefore, the second electrode 44 is disposed on the surface on which the first electrode 42 is disposed (see FIG. 4A), and the terminal bases 46a, 48a are disposed on the opposite surface (see FIG. 4B).

Before the folding process, as shown in FIG. 2, hooks 50, 52 may be fixed to the upper surfaces of the terminal bases 46a, 48a by being fitted in respective grommets 54, 56 that have been inserted in the holes 47a, 47b from the reverse surface of the base 28. The hooks 50, 52 and the grommets 54, 56 are reliably fixed to the second reference region 24b, so that the hooks 50, 52 are electrically connected to the terminal bases 46a, 48a. After reference region 24 has been folded over on itself about the fold 30, the hooks 50, 52 project upwardly, providing substantially cylindrical small-diameter pins 50a, 52a to be connected to respective connection holes 14a, 14b (see FIG. 1) of the electric energizer 14.

As shown in FIG. 2, the donor pad 32, the reference pad 36, the donor gel 34, and the reference gel 38 are placed in respective positions on the folded electrode film 20, thereby completing the patch 12.

The electric energizer 14 has on its upper surface a first LED 62 for emitting green light, a second LED 64 for emitting yellow light, and a main switch 66 for turning on the power supply of the electric energizer 14 (see FIG. 1). The connection holes 14a, 14b to which the pins 50a, 52a of the hooks 50, 52 of the patch 12 are to be connected are formed in the bottom surface of the electric energizer 14 (see FIG. 1). The electric energizer 14 houses therein an electric circuit, not shown, for applying a voltage between the connection holes 14a, 14b.

As shown in FIG. 3A, the wiring process on the electrode film 20 of the patch 12 is finished simply by forming the first electrode 42, the second electrode 44, the first connection terminal wire 46, and the second connection terminal wire 48 on one surface of the base 28. Thereafter, the electrode film 20 of desired constitution is formed simply by folding the reference region 24 over on itself about the fold 30. Therefore, the wiring process is simplified for an increased production efficiency, and the cost of the patch 12 is reduced because only one printing plate is required to print the wires on one side of the base 28. As with the patch 12, the wires on the donor region 22 to be kept in intimate contact with the patient's arm can be of a flexible wiring structure, allowing the donor section 16 to be held in sufficiently intimate with the patient.

Of the gels that function as a pair of electrodes to be applied to the skin of the patient, the donor gel 34 which holds a drug is provided on the donor section 16 on which the electric energizer 14 is not placed. Consequently, the flexibility of the donor section 16 is not impaired by the electric energizer 14. Even if the blood vessel in the region of the hemodialysis patient to be punctured (a shunt created in the arm) is significantly raised, the donor section 16 containing the drug can easily be elastically deformed and securely held in intimate contact with that region of the hemodialysis patient. Since the donor section 16 and the reference section 18 are spaced from each other with the bridge region 26 interposed therebetween, the donor section 16 is made highly flexible for higher freedom with which the patch 12 is applied to the patient. In addition, as the electric energizer 14 is directly connected to the patch 12, the device 10 as a whole is small in size and can be handled with ease.

Operation of the device 10 will briefly be described below. Before administering the drug to the patient using the device 10, the medical service worker or patient applies the patch 12 to the region of the patient through which the drug is to be delivered. The patch 12 needs to be applied to the skin of the patient so that the donor gel 34 covers the region of the patient through which the drug is to be delivered. Since the gap 43 passes applied light therethrough, the medical service worker or patient can visually recognize the region of the patient through the gap 43 from the upper surface of the patch 12, i.e., the surface of the patch 12 opposite to the surface on which the donor gel 34 is disposed, or in other words, the surface opposite to the contact surface. Accordingly, the medical service worker or patient can apply the patch 12 so that the donor gel 34 is held in contact with the region of the patient. Furthermore, inasmuch as the straight wires 42d, 42e that extend through the centers of the concentric circular wires 42a, 42b, 42c and are oriented perpendicularly to each other function as aiming lines (a reticle or an aim) for indicating the center of the first electrode 42, the straight wires 42d, 42e help the medical service worker or patient to apply the patch 12 so that the center of the donor gel 34 is held in contact with the region of the patient.

After having applied the patch 12 to the skin of the patient, the medical service worker or patient places the electric energizer 14 on the upper surface of the patch 12 in a manner to connect the hooks 50, 52 of the patch 12 to the connection holes 14a, 14b of the electric energizer 14, and then switches on the electric energizer 14, i.e., turns on the main switch 66 of the electric energizer 14. The electric energizer 14 energizes the patch 12 to start delivering the drug sealed in the donor gel 34 into the patient. Specifically, when the main switch 66 is turned on, the hook (first connection terminal) 50 connected to the connection hole (third connection terminal) 14a of the electric energizer 14 supplies an electric current through the terminal base 46a, the connection wire 46b, and the first electrode 42 of the patch 12 to the donor gel 34. The electric current supplied to the donor gel 34 is then introduced into the body (external conductor) of the patient, and flows through the reference gel 38, the second electrode 44, the electrode pad 48c, the connection wire 48b, the terminal base 48a, and the hook (second connection terminal) 52 of the patch 12, back to the electric energizer 14 via the connection hole (fourth connection terminal) 14b. The drug is delivered with the electric current into the patient.

The energization of the patch 12 by the electric energizer 14 is automatically finished a predetermined period of time after the electric energizer 14 started to energize the patch 12. If the energization of the patch 12 by the electric energizer 14 is to be finished early before the predetermined period of time elapses, then the medical service worker or patient turns off the main switch 66 to forcibly stop the electric energizer 14 from energizing the patch 12. The main switch 66 should preferably be positioned on the inside of the patch 12, i.e., more closely to the donor gel 34 than the reference gel 38. When a drug is administered to a hemodialysis patient by puncture, it is the usual practice to deliver the drug simultaneously through a plurality of locations into the hemodialysis patient, using a plurality of transdermal drug delivery devices. If the main switch 66 is positioned more remotely from the donor gel 34 than the reference gel 38 on the patch 12 of each of the transdermal drug delivery devices, then the main switch 66 of one of the transdermal drug delivery devices may be positioned in the vicinity of the main switch 66 of another one of the transdermal drug delivery devices, and may not smoothly be operated.

While the electric energizer 14 is energizing the patch 12, if the value of resistance between the donor gel 34 and the reference gel 38 falls within a predetermined range, then the electric energizer 14 assesses the device 10 as operating in a normal state, and turns on the first LED 62 to emit light. If the value of resistance between the donor gel 34 and the reference gel 38 falls outside of the predetermined range, then the electric energizer 14 assesses the device 10 as being in an abnormal state, and turns on the second LED 64 to emit light. When the value of resistance between the donor gel 34 and the reference gel 38 falls outside of the predetermined range, the drug cannot be delivered appropriately, and hence the electric energizer 14 turns on the second LED 64 to warn the user. The value of resistance between the donor gel 34 and the reference gel 38 may fall outside of the predetermined range due to a contact failure between the gels and the skin, a short-circuit on a wet skin, a connection failure between the device 10 and the patch 12, a short-circuit within the patch 12, etc. The electric energizer 14 may also produce a warning sound in addition to the LED light emission.

### [Modification 1]

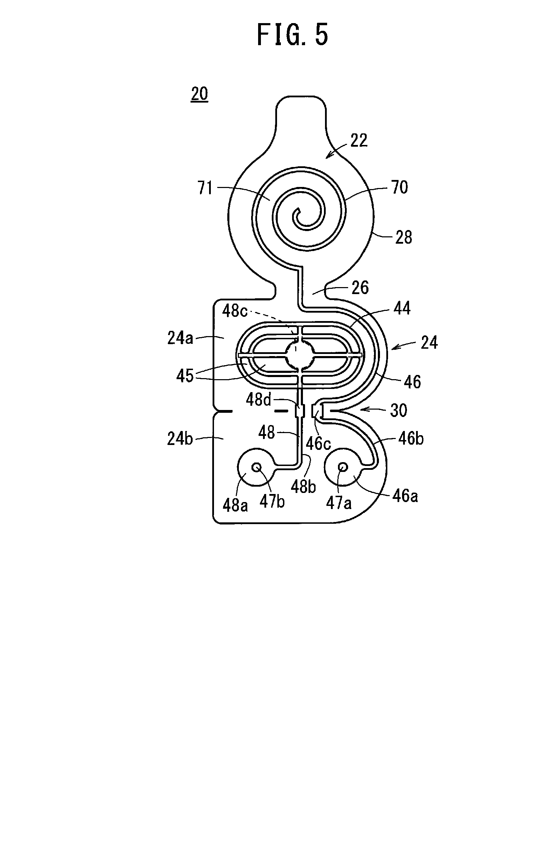

The above embodiment may be modified as follows: FIG. 5 is a bottom view of an electrode film 20 according to Modification 1 before a reference region 24 thereof is folded over on itself. Those parts of the electrode film 20 according to Modification 1 which are identical to those of the above embodiment are denoted by identical reference characters, and only different parts will be described below. In the above embodiment, the printed pattern of the first electrode 42 includes the three concentric circular wires 42a, 42b, 42c and the two straight wires 42d, 42e extending through the centers of the concentric circular wires 42a, 42b, 42c and oriented perpendicularly to each other. According to Modification 1, the printed pattern of a first electrode 70 is made up of a helical wire extending helically radially outwardly from its center. The printed pattern of the first electrode 70 has a helical gap 71 that allows the medical service worker or patient to visually recognize through the helical gap 71 the region of the patient to which the drug is to be delivered, from the upper surface of the patch 12. Accordingly, the medical service worker or patient can apply the patch 12 so that the donor gel 34 is held in contact with the region of the patient.

Since the spiral printed pattern of the first electrode 70 extends spirally radially outwardly from its center, the medical service worker or patient is able to easily recognize the center of the first electrode 70, and hence can apply the patch 12 so that the center of the donor gel 34 is held in contact with the region of the patient.

### [Modification 2]

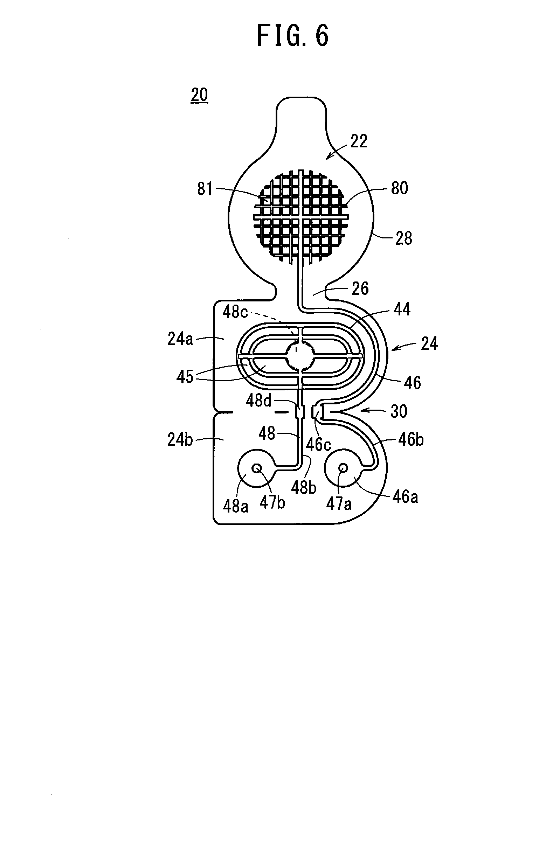

The above embodiment may also be modified as follows: FIG. 6 is a bottom view of an electrode film 20 according to Modification 2 before a reference region 24 thereof is folded over on itself. Those parts of the electrode film 20 according to Modification 2 which are identical to those of the above embodiment are denoted by identical reference characters, and only different parts will be described below. According to Modification 2, the printed pattern of a first electrode 80 is of a mesh shape including two straight wires extending perpendicularly to each other and a plurality of straight wires having a different thickness than the two straight wires and extending parallel to the two straight wires. The printed pattern of the first electrode 80 has gaps 81 between the straight wires in the mesh shape, and the gaps 81 allow the medical service worker or patient to visually recognize through the gaps 81 the region of the patient to which the drug is to be delivered, from the upper surface of the patch 12. Accordingly, the medical service worker or patient can apply the patch 12 so that the donor gel 34 is held in contact with the region of the patient.

Since the two straight wires that extend through the center of the first electrode 80 are different in thickness than the other straight wires of the mesh shape, the medical service worker or patient is able to easily recognize the center of the first electrode 80, and hence can apply the patch 12 so that the center of the donor gel 34 is held in contact with the region of the patient.

### [Modification 3]

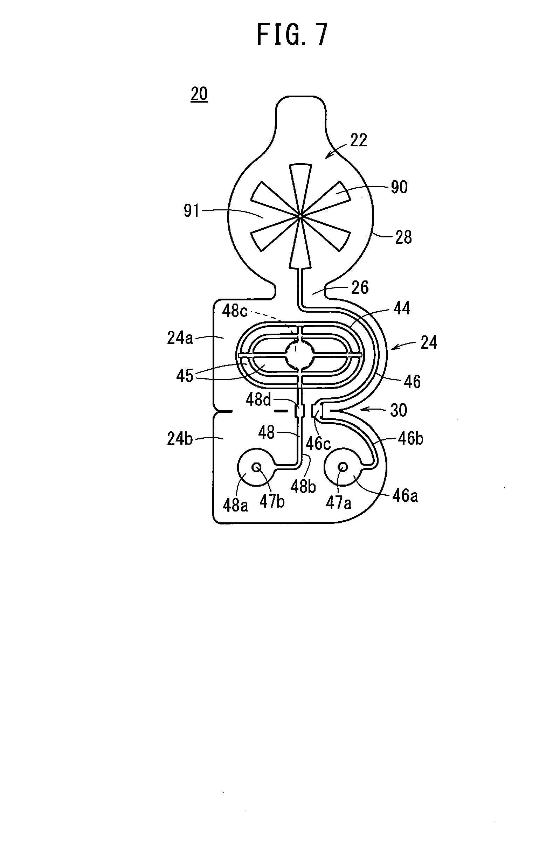

The above embodiment may further be modified as follows: FIG. 7 is a bottom view of an electrode film 20 according to Modification 3 before a reference region 24 thereof is folded over on itself. Those parts of the electrode film 20 according to Modification 3 which are identical to those of the above embodiment are denoted by identical reference characters, and only different parts will be described below. According to Modification 3, the printed pattern of a first electrode 90 is made up of a radial shape extending radially outwardly from its center. The radial shape is made of a plurality of, ranging from 4 to 8, sectorial shapes which have respective pointed vertexes positioned at the center of the first electrode 90. The printed pattern of the first electrode 90 has radial gaps 91 between the sectorial shapes, and the gaps 91 allow the medical service worker or patient to visually recognize through the gaps 91 the region of the patient to which the drug is to be delivered, from the upper surface of the patch 12. Accordingly, the medical service worker or patient can apply the patch 12 so that the donor gel 34 is held in contact with the region of the patient.

Since the radial printed pattern of the first electrode 90 extends radially outwardly from its center, the medical service worker or patient is able to easily recognize the center of the first electrode 90, and hence can apply the patch 12 so that the center of the donor gel 34 is held in contact with the region of the patient.

Although the preferred embodiments of the present invention have been described above, the technical scope of the present invention is not limited to the description of the above embodiments. It is obvious to those skilled in the art that various changes and modifications can be made to the above embodiments. It is apparent from the scope of the appended claims that such changes and modifications are covered by the technical scope of the present invention.

## Claims

1. An iontophoresis patch (12) including a first contact member (34) and a second contact member (38) configured to be held in contact with an external conductor and configured to supply an electric current from an electric energizer (14) to the external conductor, comprising:
a donor section (16) including the first contact member (34), the first contact member (34) containing a drug to penetrate into the external conductor;
a reference section (18) including the second contact member (38), the reference section (18) being placed on the external conductor and being spaced from the donor section (16); and
an electrode body (20) having a first electrode (42, 70, 80, 90) and a second electrode (44) configured to supply an electric current from the electric energizer (14) to the first contact member (34) and second contact member (38), the electrode body (20) extending over the donor section (16) and the reference section (18);
wherein the electrode body (20) is of a one-sided wiring structure having the first electrode (42, 70, 80, 90) and the second electrode (44) which are formed by printing on one surface of a flexible film (28) in an electrically conductive ink;
the first electrode (42, 70, 80, 90) has a printed pattern including a gap (43, 71, 81, 91) which is devoid of the electrically conductive ink; and
the flexible film (28) and the first contact member (34) are transparent.

2. The iontophoresis patch (12) according to claim 1, wherein the printed pattern of the first electrode (42) is of a shape having at least either concentric circular wires (42a, 42b, 42c) or straight wires (42d, 42e) extending centrally through the donor section (16), the straight wires (42d, 42e) functioning as aiming lines for indicating a center of the first electrode (42).

3. The iontophoresis patch (12) according to claim 1, wherein the printed pattern of the first electrode (70) is of a spiral shape.

4. The iontophoresis patch (12) according to claim 1, wherein the printed pattern of the first electrode (80) is of at least a mesh shape.

5. The iontophoresis patch (12) according to claim 1, wherein the printed pattern of the first electrode (90) is of a radial shape.
